# EUROPÄISCHE PATENTSCHRIFT

(11) **EP 0 957 165 B1**
(45) Veröffentlichungstag und Bekanntmachung des Hinweises auf die Patenterteilung: **18.04.2007**
(21) Anmeldenummer: 99112607.9
(22) Anmeldetag: 01.12.1998
(51) Int. Cl.: C12N 15/12, C12N 15/65, C12N 15/67, C12N 15/85, C12N 15/90, C12N 5/10, C07K 14/505, C12Q 1/68

(54) **Optimierung von Zellen für die endogene Genaktivierung**
Optimizing cells for endogenous gene activation
Optimalisation de cellules pour l'activation de l'expression de gènes endogènes

(30) Priorität: 01.12.1997 EP 97121075
(43) Veröffentlichungstag der Anmeldung: 17.11.1999
(62) Teilanmeldung aus: 98122807.5
(73) Patentinhaber: Roche Diagnostics GmbH, 68305 Mannheim (DE)
(72) Erfinder: Honold, Konrad, 82377 Penzberg (DE); Holtschke, Thomas, 81369 München (DE); Stern, Anne, 82377 Penzberg (DE)
(74) Vertreter: Weiss, Wolfgang

(56) Entgegenhaltungen:
- EP-A- 0 343 783
- EP-A- 0 747 485
- WO-A-90/11354
- WO-A-91/09955
- WO-A-96/29411
- WO-A-96/39426
- SEMENZA G L ET AL: "A NUCLEAR FACTOR INDUCED BY HYPOXIA VIA DE NOVO PROTEIN SYNTHESIS BINDS TO THE HUMAN ERYTHROPOIETIN GENE ENHANCER AT A SITE REQUIRED FOR TRANSCRIPTIONAL ACTIVATION" MOLECULAR AND CELLULAR BIOLOGY, Bd. 12, Nr. 12, 1. Dezember 1992 (1992-12-01), Seiten 5447-5454, XP000562137 ISSN: 0270-7306
- DAMERT A ET AL: "Activator-protein-1 binding potentiates the hypoxia -induciblefactor-1-mediated hypoxia-induced transcriptional activation of vascular-endothelial growth factor expression in C6 glioma cells" BIOCHEMICAL JOURNAL, Bd. 327, Nr. PART 02, 15. Oktober 1997 (1997-10-15), Seiten 419-423, XP002083936 ISSN: 0264-6021
- TIAN ET AL: "Endothelial PAS domain protein 1 (EPAS1), a transcription factor selectively expressed in endothelial cells" GENES AND DEVELOPMENT, Bd. 11, Nr. 1, 1. Januar 1997 (1997-01-01), Seiten 72-82 82, XP002100643 ISSN: 0890-9369
- SEMENZA G L ET AL: "TRANSCRIPTIONAL REGULATION OF GENES ENCODING GLYCOLYTIC ENZYMES BY HYPOXIA-INDUCIBLE FACTOR 1" JOURNAL OF BIOLOGICAL CHEMISTRY, Bd. 269, Nr. 38, 23. September 1994 (1994-09-23), Seiten 23757-23763, XP000578184 ISSN: 0021-9258
- WANG G L ET AL: "GENERAL INVOLVEMENT OF HYPOXIA-INDUCIBLE FACTOR 1 IN TRNASCRIPTIONAL RESPONSE TO HYPOXIA" PROCEEDINGS OF THE NATIONAL ACADEMY OF SCIENCES OF USA, Bd. 90, Nr. 9, 1. Mai 1993 (1993-05-01), Seiten 4304-4308, XP000561804 ISSN: 0027-8424
- G U DACHS ET AL: "Targeting gene expression to hypoxic tumor cells" NATURE MEDICINE, Bd. 3, Nr. 5, 1. Mai 1997 (1997-05-01), Seiten 515-520, XP002096343 ISSN: 1078-8956
- DATABASE WPI Section Ch, Week 198801 Derwent Publications Ltd., London, GB; Class B04, AN 1988-003020 XP002118545 & JP 62 265992 A (AJINOMOTO KK), 18. November 1987 (1987-11-18)

## Beschreibung

Die Erfindung betrifft das Einführen zweier Nukleinsäuresequenzen, an die das Aktivatorprotein Hypoxia-Inducible-Factor (HIF) bindet, in das Genom einer eukaryontischen Zelle durch homologe Rekombination, um die Expression eines Zielgens zu verändern.

Die Genexpression in einer Zelle kann konstitutiv, beispielsweise bei sogenannten Housekeeping-Genen, oder reguliert erfolgen. Die regulierte Expression ist insbesondere für Gene notwendig, die nur in einem bestimmten Entwicklungsstadium der Zelle oder bei einer Änderung der Umweltbedingungen exprimiert werden müssen.

Die Expression wird auf der Transkriptionsebene durch den operativ mit der kodierenden Nukleinsäuresequenz verbundenen Promotor reguliert, dessen Aktivität durch Repressoren und Aktivatoren gesteuert werden kann. Eine Bindung von Repressoren bzw. Aktivatoren an nichtkodierende Nukleinsäuresequenzen des Gens kann eine Verminderung bzw. Erhöhung der Aktiviät des Promotors bewirken (L. Stryer, Biochemie, Kapitel 12, Spektrum der Wissenschaft, Verlagsgesellschaft, Heidelberg, 1990). Die Menge der in einer Zelle enthaltenen Repressoren bzw. Aktivatoren wird wiederum durch Faktoren, wie beispielsweise Umweltbedingungen, reguliert. Ein Beispiel für Aktivatoren sind die Hypoxia-Inducible-Factoren (HIF), die durch vermindertes O₂-Angebot induziert werden und zu einer erhöhten Expression des Erythropoietingens führt (Blanchard K.L. et al., Hypoxic induction of the human erythropoietin gene: Cooperation between the promotor and enhancer, each of which contains steroid receptor response elements, (1992), Mol.Cell.Biol. 12, 5373-5385; Wang G.L. and Semenza G.L., Characterization of hypoxia-inducible factor 1 and regulation of DNA binding activity by hypoxia, (1993), J.Biol.Chem., 268, 21513-21518; Wang G.L. et al., Hypoxia-inducible factor 1 is a basic-helix-loop-helix-PAS heterodimer regulated by cellular O₂ tension, (1995), Proc.Natl.Acad.Sci. USA, 92, 5510-5514).

Desweiteren ist die Menge eines exprimierten Proteins von der Stabilität der mRNA abhängig. Im 3'-seitigen Bereich einer mRNA sind Erkennungssequenzen für mRNA abbauende Enzyme lokalisiert, die die Stabilität der mRNA und somit die Expressionshöhe beeinflussen (Shaw G. and Kamen R., A Conserved AU Sequence from the 3'Untranslated Region of GM-CSF mRNA Mediates Selective mRNA Degradation, Cell (1986), 659-667). Die Halbwertszeit der mRNA korreliert dabei mit der Menge exprimierten Proteins. Eine dritte Ebene der Expressionsregulation ist die Translation.

Die Expression eines Gens unterliegt somit komplexen Regulationsmechanismen, die im Einzelfall sehr unterschiedlich sein können.

Proteine können mit Hilfe der rekombinanten DNA-Technologie gewonnen werden, welche die Kenntnisse der Expressionsregulation nutzt (Sambrook et al., 1989 Molecular Cloning, A Laboratory Manual, Cold Spring Harbor). Hierzu werden Vektoren verwendet, die eine das entsprechende Protein kodierende Nukleinsäuresequenz unter Kontrolle eines geeigneten Promotors enthalten, sowie weitere für die Expression des Proteins und zur Replikation des Vektors notwendige Sequenzen. Der Vektor wird dann mittels bekannter Verfahren in eine Wirtszelle eingebracht, die Zelle wird kultiviert und das rekombinante Protein kann aus der Zelle oder dem Kulturmedium gewonnen werden.

Als Wirtszelle können prokaryontische oder eukaryontische Zellen verwendet werden. Prokaryontische Zellen, insbesondere E.coli-Zellen, sind in ihrer Handhabung unproblematisch, weisen aber bei einer rekombinanten Expression von eukaryontischen Proteinen eine Reihe von Nachteilen auf.

Prokaryonten und Eukaryonten unterscheiden sich im Expressionsprozessierungsweg, in den Zellmilieu-Bedingungen sowie in den bei der Proteinprozessierung beteiligten Chaperons. Deshalb können in einem in Prokaryonten hergestellten eukaryontischen Protein entscheidende Unterschiede im Vergleich zu dem entsprechenden nativen Protein auftreten. Beispielsweise kann das Proteinfaltungsmuster und die Aktivität des Proteins verändert sein. Auch werden Proteine in einer prokaryontischen Wirtszelle in der Regel nicht glycosyliert. Ein korrektes Glycosylierungsmuster stellt aber in vielen Fällen, beispielsweise bei der Herstellung von Proteinen für eine pharmazeutische Formulierung, ein entscheidendes Merkmal für die Wirksamkeit und Verträglichkeit dar.

Glycosylierte Proteine werden deshalb mittels eukaryontischer Wirtszellen oder Zellinien, beispielsweise CHO (Chinese Hamster Ovary) Zellen, hergestellt. Trotz der Verwendung eukaryontischer Zellen können aufgrund von Speziesunterschieden, beispielsweise bei der Expression eines humanen Proteines in nichthumanen Zellen, Veränderungen in dem rekombinant hergestellten Protein auftreten, wodurch dieses für viele Anwendungen unbrauchbar wird.

Zur rekombinanten Herstellung von Proteinen werden Wirtszellen transient oder stabil mit Expressionsvektoren transfiziert, wobei insbesondere bei großtechnischen Herstellungsverfahren stabil transfizierte Zellen verwendet werden.

Die unspezifische, zufällige Integration der Expressionsvektorsequenzen in das Genom der Wirtszelle kann zu Zellen mit geringer Produktionsleistung oder instabilen Eigenschaften der Zellen führen. Beispielsweise kann im Laufe des Produktionsprozesses die Produktionsleistung sinken oder die Fähigkeit der Zellen das rekombinante Protein zu exprimieren geht ganz verloren.

Die vorliegende Erfindung betrifft ein Verfahren zum Verändern der Expression einer in einer humanen Zelle endogen vorliegenden Nukleinsäuresequenz,
dadurch gekennzeichnet,
dass
(a) die Zelle transfiziert wird mit einem Vektor, umfassend
   (i) zwei des Aktivatorprotein HIF bindende Nukleinsäuresequenzen, ausgewählt aus der Sequenz gemäß SEQ ID NO 1 und der Sequenz gemäß SEQ ID NO 2
   (ii) ein positives Selektionsmarkergen,
   (iii) die Sequenzen (i) und (ii) flankierende DNA-Sequenzen, die homolog zu einem Nukleinsäureabschnitt im Genom der Zelle sind, wobei die homologen Sequenzen (iii) so gewählt sind, dass eine genomische Integration der Aktivatorprotein-bindenden Nukleinsäuresequenzen (i) im Bereich der Expressionskontrollsequenz des Zielgens erfolgt, um eine homologe Rekombination zu erlauben,
(b) die transfizierte Zelle unter Bedingungen kultiviert wird, unter denen eine homologe Rekombination des Vektors erfolgt,
(c) die gemäß Schritt (b) erhaltene Zelle gewonnen wird, und
(d) die Zelle transfiziert wird mit einem Vektor, umfassend
   (i) eine für das Aktivatorprotein HIF-102 und eine für des Aktivatorprotein HIF-1B kodierende Nukleinsauresequenz, die operativ verbunden sind mit einer in dieser Zelle aktiven Expressionskontrollsequenz, und
   (ii) gegebenenfalls ein positives Selektionsmarkergen.

Durch genomische Integration einer Nukleinsäuresequenz, welche die Bindung eines oder mehrerer Aktivatorproteine (durch Bindung an die Nukleinsäuresequenz die Genexpression erhöhende Proteine), im Bereich der Expressionskontrollsequenz eines Zielgens, insbesondere in deren regulatorischen Bereichen, wird die Expression des Zielgens überraschenderweise nicht verringert, sondern es wird im Gegensatz dazu sogar möglich, durch geeignete Kulturbedingungen die Expression des endogen vorliegenden Zielgens zu erhöhen bzw. die Expression eines nichtexprimierten endogen vorliegenden Zielgens zu induzieren.

Aktivatorproteine sind die Hypoxia-Inducible-Faktoren HIF-1α und HIF-1β.

Nach operativer Verknüpfung zweier HIF bindenden Nukleinsäuresequenzen mit einem endogen vorliegenden Zielgen kann bei Auswahl geeigneter Kulturbedingungen die Expression des Zielgens reguliert werden. Dies bietet insbesondere bei einer großtechnischen Herstellung den Vorteil, daß die Expression eines Proteins zu einem für den Herstellungsprozeß optimalen Zeitpunkt induziert werden kann. Vorteilhaft ist dabei, daß die durchschnittliche Verweildauer des Syntheseproduktes im Kulturmediumüberstand verringert wird. Dadurch kann auch die Menge an unerwünschten Abbauprodukten des Proteins verringert werden. Dies wirkt sich positiv bei der Durchführung deranschließenden Reinigungsschritte aus, reduziert die Herstellungskosten und führt zu einem qualitativ verbesserten Endprodukt.

Zur Durchführung des erfindungsgemäßen Verfahrens genügt es zwei HIF-bindende Nukleinsäuresequenzen ausgewählt aus der 53 bp Sequenz gemäß Sequenz ID NO. 1, der 43 bp Sequenz gemäß Sequenz ID NO. 2 mit dem Zielgen operativ zu verknüpfen.

Die Verwendung von zwei HIF-bindenden Nukleinsäuresequenzen führt überraschenderweise zu einer synergistischen Wirkung. Dadurch wird eine stärkere Erhöhung der Expression endogener Nukleinsäuren erreicht, als bei Verwendung jeder dieser Sequenzen alleine.

Die mit der für das Aktivatorprotein kodierenden Nukleinsäuresequenz operativ verbundene Expressionskontrollsequenz kann induzierbar sein, so daß eine zusätzliche Möglichkeit der Aktivierung durch geeignete Kulturbedingungen, wie z.B. Zugabe von Hormonen oder Schwermetallen, erreicht werden kann. Dadurch wird es möglich, die Expression eines endogenen Zielgens zu einem für den Herstellungsprozeß optimalen Zeitpunkt zu induzieren.

Die Verwendung einer konstitutiv aktiven Expressionskontrollsequenz hat den Vorteil, daß das Aktivatorprotein unabhängig von der Zugabe von Aktivatoren in das Kulturmedium konstitutiv exprimiert wird.

Wenn die Aktivatorprotein-bindende Nukleinsäuresequenz eine HIF-bindende Nukleinsäuresequenz ist, kann beispielsweise die Expression des Zielgens durch geeignete Kulturbedingungen, z.B. bei einer O₂-Konzentration von 0,1 - 2% induziert werden.

Noch ein weiterer Gegenstand der vorliegenden Erfindung ist eine humane Zelle, die erhältlich ist nach einem wie oben beschriebenen Verfahren. Diese Zelle ist dadurch gekennzeichnet dass sie mindestens ein endogen in der Zelle vorliegenden Gen operativ verknüpft mit einer Expressionskontrollsequenz und mit zwei heterologen, chromosomal lokalisierten, im Bereich der Expressionskontrollsequenz eines Zielgens genomisch integrierten, das Aktivatorprotein HIF bindenden Nukleinsäurefragmente ausgewählt aus der Sequenz gemäß SEQ ID NO. 1 und der Sequenz gemäß SEQ ID NO. 2 enthält, und einen Vektor, umfassend eine für das Aktivatorprotein HIF-1α und eine für das Aktivatorprotein HIF-1β Kodierende Nukleinsäuresequence, die operativ verbunden sind mit einer in dieser Zelle aktiven ExpressionsKontrollsequenz, und gegebenenfalls ein positives Selektionsmarkergen enthält. Mit Hilfe eines ortsspezifischen Rekombinationssystems können Aktivatorprotein-bindende Nukleinsäurefragmente im Genom ausgetauscht werden, so daß eine einfache Identifizierung einer für ein bestimmtes Zielgen optimalen Aktivatorsequenz möglich wird.

Die Erfindung wird durch das nachfolgende Beispiel, Figur und das Sequenzprotokoll erläutert.

### Figurenbeschreibung

### Figur 1

zeigt die CMV-Promotor/HIF-kontrollierte Erythropoietin (EPO)-Expression von HeLa S3 Zellen, die transfiziert wurden mit den Vektoren pHYG, pHIF-1α und pARNT (pHIF-1β) und deren EPO-Expression 3, 4 und 5 Tage nach der Transfektion in den Zellüberständen gemessen wurde (Erythropoietinkonzentration in µg/ml).

pHYG: Kontrollvektor, pHIF-1α: eine HIF-1α cDNA unter Kontrolle eines SRα-Promotors, pARNT: eine HIF-β cDNA unter Kontrolle eines CMV-Promotors.
SEQ ID NO. 1 zeigt eine erste HIF-bindende Nucleotidsequenz,
SEQ ID NO. 2 zeigt eine zweite HIF-bindende Nuceotidsequenz,
SEQ ID NO. 3 zeigt eine loxP-Sequenz.

### BEISPIELE

### Beispiel 1 Expression eines Erythropoietin-Gens unter Kontrolle eines CMV-Promotors und Überexpression von HIF

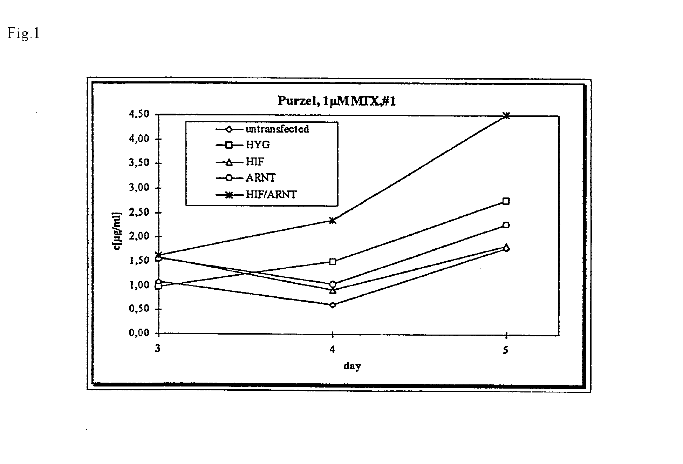

Die Vektoren pHYG, pHIF-1α und pARNT (vgl. Figur 3) werden in genetisch veränderte HeLa-S3 Zellen transfiziert. In den Zellen wurde proximal zu dem Erythropoietin-Gen (EPO)-Translationsstart eines EPO-Allels ein CMV (Cytomegalovirus) Promotor eingeführt, der die EPO-Expression kontrolliert. Die Zellen produzieren normalerweise 1 *µ*g Erythropoietin pro 24 Stunden pro 10⁷ Zellen. Sie werden 24 Stunden vor der Transfektion mit einer Konzentration von 6 x 10⁴ Zellen pro 6 Lochplatte passagiert. Am Tag der Transfektion werden die Zellen mit einem DNA-DOTAP-Gemisch inkubiert. Das Gemisch enthält 1,25 µg des jeweiligen Vektors, 10µl DOTAP (Boehringer Mannheim 1202375) ad 75 µl in 20 mM Hepespuffer pro Loch. Das Gemisch wird für 10-15 Minuten bei Raumtemperatur vorinkubiert. Die Zellen werden dann in 3 ml Medium pro Loch mit dem DNA-DOTAP für 6 Stunden inkubiert. Anschließend werden die Zellen zweimal mit PBS-Puffer gewaschen und in Vollmedium für 5 Tage kultiviert. Am Tag 3, 4 und 5 werden jeweils 100 *µ*l Überstand entnommen und mit einem Erythropoietin ELISA analysiert. Der Assay ist am Tag 5 abgeschlossen und die Zellzahl wird bestimmt. Die Erythropoietinmenge pro Loch wird bezogen auf gleiche Zellzahl berechnet (vgl. Figur 3).

Das Beispiel zeigt, daß eine Induktion des Erythropoietingens durch HIF immer noch möglich ist, obwohl eine heterologe Expressionskontrollsequenz (CMV-Promotor) in die Promotorregion eines Allels des Erythropoietingens eingeführt wurde. Die gemessene Erhöhung der Erythropoietinkonzentration deutet auf eine synergistische Wirkung des Hypoxia-induzierten Faktors bzw. der Hypoxia-induzierten Faktoren auf beide Allele hin.

Es wird somit deutlich, daß die Expression einer endogenen Nukleinsäuresequenz durch Einführen einer heterologen Expressionskontrollsequenz erhöht werden kann. Wird ein Aktivator (HIF) in der Zelle exprimiert, für den in der Expressions-kontrollsequenz bindende Nukleinsäuresequenz vorhanden sind, so kann die Expression dieses Gens weiter gesteigert werden. Sind entsprechende Sequenzen in diesem Genlocus nicht vorhanden, können sie gezielt durch das erfindungsgemäße Verfahren mittels homologer Rekombination in das Genom eingebracht werden.

## Patentansprüche

1. Verfahren zum Verändern der Expression einer in einer humanen Zelle endogen vorliegenden Nukleinsäuresequenz,
**dadurch gekennzeichnet,**
**dass**
(a) die Zelle transfiziert wird mit einem Vektor, umfassend
(i) zwei des Aktivatorprotein HIF bindende Nukleinsäuresequenzen, ausgewählt aus der Sequenz gemäß SEQ ID NO. 1 und der Sequenz gemäß SEQ ID NO. 2
(ii) ein positives Selektionsmarkergen,
(iii) die Sequenzen (i) und (ii) flankierende DNA-Sequenzen, die homolog zu einem Nukleinsäureabschnitt im Genom der Zelle sind, wobei die homologen Sequenzen (iii) so gewählt sind, dass eine genomische Integration der Aktivatorprotein-bindenden Nukleinsäuresequenzen (i) im Bereich der Expressionskontrollsequenz des Zielgens erfolgt, um eine homologe Rekombination zu erlauben,
(b) die transfizierte Zelle unter Bedingungen kultiviert wird, unter denen eine homologe Rekombination des Vektors erfolgt,
(c) die gemäß Schritt (b) erhaltene Zelle gewonnen wird, und
(d) die Zelle transfiziert wird mit einem Vektor, umfassend
(i) eine für das Aktivatorprotein HIF-102 und eine für des Aktivatorprotein HIF-1B kodierende Nukleinsauresequenz, die operativ verbunden sind mit einer in dieser Zelle aktiven Expressionskontrollsequenz, und
(ii) gegebenenfalls ein positives Selektionsmarkergen.

2. Humane Zelle, erhältlich durch ein Verfahren nach Anspruch 1.

3. Humane Zelle,
**dadurch gekennzeichnet,**
**dass** sie mindestens ein endogen in der Zelle vorliegenden Gen operativ verknüpft mit einer Expressionskontrollsequenz und mit zwei heterologen, chromosomal lokalisierten, im Bereich der Expressionskontrollsequenz eines Zielgens genomisch integrierten, das Aktivatorprotein HIF bindenden Nukleinsäurefragmente ausgewählt aus der Sequenz gemäß SEQ ID NO. 1 und der Sequenz gemäß SEQ ID NO. 2 enthält, und einen Vektor, umfassend eine für das Aktivatorprotein HIF-1α und eine für das Aktivatorprotein HIF-1β Kodierende Nukleinsäuresequence, die operativ verbunden sind mit einer in dieser Zelle aktiven ExpressionsKontrollsequenz, und gegebenenfalls ein positives Selektionsmarkergen enthält.

## Claims

1. A process for changing the expression of a nucleic acid sequence that is present endogenously in a human cell which is **characterized in that**
(a) the cell is transfected with a vector comprising
(i) two nucleic acid sequences that bind the activator protein HIF, selected from the sequence according to SEQ ID NO 1 and the sequence according to SEQ ID NO 2,
(ii) a positive selection marker gene,
(iii) DNA sequences flanking the sequences (i) and (ii) which are homologous to a nucleic acid section in the genome of the cell, wherein the homologous sequences (iii) are selected such that a genomic integration of the activator protein-binding nucleic acid sequences (i) takes place in the region of the expression control sequence of the target gene, in order to allow a homologous recombination,
(b) the transfected cell is cultured under conditions under which a homologous recombination of the vector takes place,
(c) the cell obtained according to step (b) is isolated, and
(d) the cell is transfected with a vector comprising
(i) a nucleic acid sequence coding for the activator protein HIF-1α and one coding for the activator protein HiF-1β which are operatively linked with an expression control sequence active in this cell and
(ii) optionally a positive selection marker gene

2. Human cell obtainable by a process according to claim 1.

3. Human cell
**characterized in that**
it contains at least one gene that is present endogenously in the cell operatively linked with an expression control sequence and with two heterologous, chromosomally located nucleic acid fragments selected from the sequence according to SEQ ID NO 1 and the sequence according to SEQ ID NO 2 that bind the activator protein HIF and are genomically integrated in the region of the expression control sequence of a target gene, and a vector comprising a nucleic acid sequence coding for the activator protein HIF-1α and a nucleic acid sequence coding for the activator protein HIF-1β which are operatively linked with an active expression control sequence in this cell, and optionally a positive selection marker gene.

## Revendications

1. Procédé de modification de l'expression d'une séquence d'acide nucléique se trouvant de manière endogène dans une cellule humaine, **caractérisé en ce que**
(a) on transfecte la cellule avec un vecteur comprenant
(i) deux séquences d'acides nucléiques se liant à la protéine activatrice HIF, choisies parmi la séquence conforme à la SEQ ID NO. 1 et la séquence conforme à la SEQ ID NO. 2,
(ii) un gène marqueur de sélection positive,
(iii) des séquences d'ADN flanquant les séquences (i) et (ii), qui sont homologues d'un fragment d'acide nucléique du génome de la cellule, les séquences homologues (iii) étant choisies de telle manière qu'il se produit une intégration des séquences d'acides nucléiques se liant à la protéine activatrice HIF dans la zone de la séquence de contrôle de l'expression du gène cible afin de permettre une recombinaison homologue,
(b) on cultive les cellules transfectées dans des conditions dans lesquelles il se produit une recombinaison homologue du vecteur,
(c) on récupère les cellules obtenues selon l'étape (b), et
(d) on transfecte la cellule avec un vecteur comprenant
(i) une séquence d'acide nucléique codant pour la protéine activatrice HIF-1α et pour la protéine activatrice HIF-1β en liaison opérationnelle avec une séquence de contrôle de l'expression active dans cette cellule, et
(ii) le cas échéant un gène marqueur de sélection positive

2. Cellule humaine pouvant être obtenue par un procédé selon la revendication 1.

3. Cellule humaine, **caractérisée en ce qu'**elle contient au moins un gène présent de manière endogène dans la cellule en liaison opérationnelle avec une séquence de contrôle de l'expression et avec deux fragments d'acides nucléiques hétérologues, choisis parmi la séquence conforme à la SEQ ID NO. 1 et la séquence conforme à la SEQ ID NO. 2, qui sont localisés sur un chromosome et intégrés dans le génome dans la zone de la séquence de contrôle de l'expression d'un gène cible et qui se lient à la protéine activatrice HIF-1α, et un vecteur comprenant une séquence d'acide nucléique codant pour la protéine activatrice HIF-1α et pour la protéine activatrice HIF-1β en liaison opérationnelle avec une séquence de contrôle de l'expression active dans cette cellule, et le cas échéant un gène marqueur de sélection positive.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Strasse 112-132
      (C) ORT: Mannheim
      (E) LAND: Deutschland
      (F) POSTLEITZAHL: D-68305
   (ii) BEZEICHNUNG DER ERFINDUNG: Optimierung von Zellen fuer die endogene Genaktivierung
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 53 Basenpaare
      (B) ART: Nucleotid
      (C) STRANGFORM: beides
      (D) TOPOLOGIE: linear
   (xi) SEQUENZBESCHREIBUNG: SEQ ID NO: 1:
      CCTCTCCTCT AGGCCCGTGG GGCTGGCCCT GCACCGCCGA GCTTCCCGGG ATG 53
(2) ANGABEN ZU SEQ ID NO: 2:
   (i) SEQUENZKENNZEICHEN:
      (A) LÄNGE: 43 Basenpaare
      (B) ART: Nucleotid
      (C) STRANGFORM: beides
      (D) TOPOLOGIE: linear
   (xi) SEQUENZBESCHREIBUNG: SEQ ID NO: 2:
      CTACGTGCTG TCTCACACAG CCTGTCTGAC CTCTCGACCC TAC 43
(2) ANGABEN ZU SEQ ID NO: 3:
   (i) SEQUENZKENNZEICHEN:
      (A) LÄNGE: 34 Basenpaare
      (B) ART: Nucleotid
      (C) STRANGFORM: beides
      (D) TOPOLOGIE: linear
   (xi) SEQUENZBESCHREIBUNG: SEQ ID NO: 3:
      TATTGAAGCA TATTACATAC GATATGCTTC AATA 34

### SEQUENZPROTOKOLL

<110> Roche Diagnostics GmbH
<120> Optimierung von Zellen für die endogene Genaktivierung
<130> 15964P EP-2 endogene Genaktivierung
<140> 99112607.9
   <141> 1998-12-01
<150> 97121075.2
   <151> 1997-12-01
<160> 3
<170> Patent In Ver. 2.1
<210> 1
   <211> 53
   <212> DNA
   <213> Homo sapiens
<400> 1
   cctctcctct aggcccgtgg ggctggccct gcaccgccga gcttcccggg atg 53
<210> 2
   <211> 43
   <212> DNA
   <213> Homo sapiens
<400> 2
   ctacgtgctg tctcacacag cctgtctgac ctctcgaccc tac 43
<210> 3
   <211> 34
   <212> DNA
   <213> Bacteriophage P1
<400> 3
   tattgaagca tattacatac gatatgcttc aata 34